# FASCICULE DE BREVET EUROPEEN

(11) **EP 1 399 101 B1**
(45) Date de publication et mention de la délivrance du brevet: **24.11.2004**
(21) Numéro de dépôt: 02738295.1
(22) Date de dépôt: 31.05.2002
(51) Int. Cl.: A61F 5/00

(54) **ANNEAU POUR GASTROPLASTIE**
MAGENBAND
GASTRIC BAND

(30) Priorité: 01.06.2001 FR 0107416
(43) Date de publication de la demande: 24.03.2004
(73) Titulaire: I.O.C., 42000 Saint-Etienne (FR)
(72) Inventeur: MOUTON, Didier, F-42270 Saint Priest en Jarez (FR)
(74) Mandataire: Myon, Gérard
(86) Numéro de dépôt international: PCT/FR2002/001848
(87) Numéro de publication internationale: WO 2002/096326

(56) Documents cités:
- EP-A- 1 036 545
- WO-A-94/27504
- DE-A- 19 751 733
- DE-U- 9 014 048
- FR-A- 2 799 118
- US-A- 4 592 339
- US-A- 6 102 922

## Description

L'invention se rattache au secteur technique des anneaux gastriques et moyens similaires susceptibles d'entourer une partie d'estomac pour assurer un effet de rétrécissement de l'estomac à un endroit donné dans le cadre du traitement de l'obésité, et ce après échec des thérapeutiques médicales telles que régimes alimentaires, suivis par nutritionnistes, programmes d'exercices physiques, cures d'amaigrissement, et critères de l'ANAES (BMI et Comorbidité).

On connaît, selon l'art antérieur, différents types d'anneaux gastriques qui sont par ailleurs associés à d'autres moyens, tels que des ballons dilatables par fluide, comme décrit dans DE-U-9724127 ou WO-A-9427504, ou en association avec un tube à flux lumineux, comme décrit dans US-A-4696288. Ces technologies sont coûteuses à mettre en oeuvre. La pose de ces anneaux reste aléatoire par l'effet du glissement de l'anneau sur l'estomac, et surtout lorsqu'il existe des hernies hiatales associées.

Un des problèmes posés à l'origine de l'invention réside dans la mise en oeuvre de la fermeture de l'anneau qui n'est pas toujours facile à exécuter et dans la sécurité de la liaison.

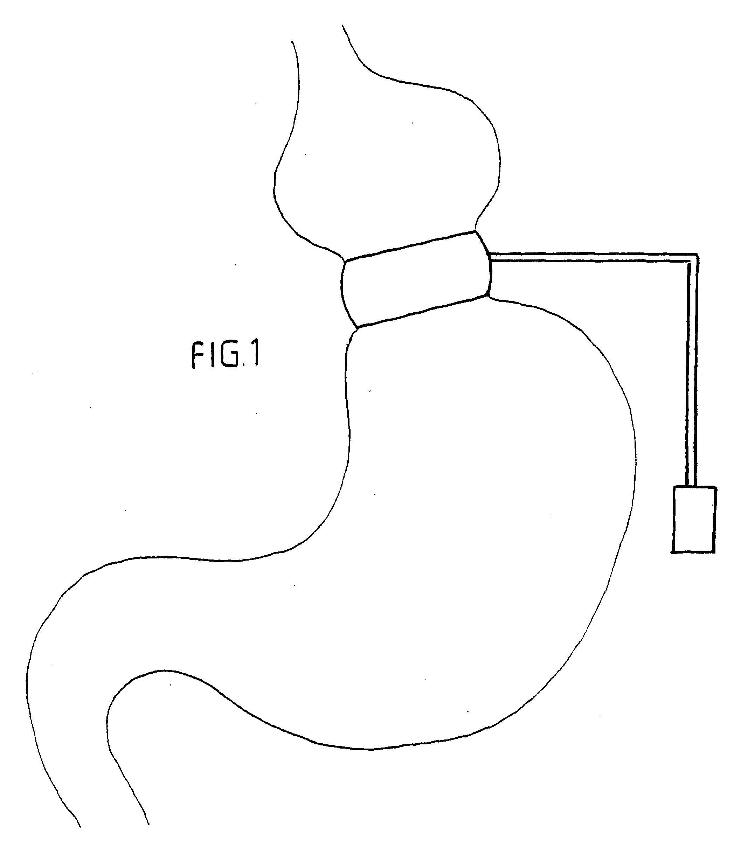

Un autre problème est la nécessité d'un contrôle du gonflage du ballon des anneaux connus par un système externe vulnérable qui gêne la majorité des opérés. Ce système inclut l'utilisation et la tenue d'un boîtier sous cutané, avec risque de contamination par l'aiguille. Cela implique des précautions spécifiques. En outre, ces anneaux sont particulièrement gênants pour le patient, en étant source de douleurs, de fuite, car il y a des risques de cassure du cheminement, le gonflage du ballon impliquant une pression. De plus, la surface interne des anneaux à ballon dilatable est parfois irrégulière lorsque le ballon n'est pas dilaté, d'où un risque de lésion ou d'irritation de la paroi gastrique. On a représenté, en figure 1, un tel anneau gastrique. La pose de ces anneaux gastriques telle que décrite ci-avant s'effectue dans le cadre d'une gastroplastie verticale.

On connaît aussi l'utilisation de bandelettes disposées dans un plan globalement horizontal. Cependant, cette technique présente aussi des inconvénients à savoir : ces bandelettes réalisées par exemple en treillis de polypropylène provoquent des incrustations de matière dans la paroi de l'estomac, avec un effet néfaste empêchant l'ablation du bandage ainsi réalisé en cas de décision de réversibilité (création d'un tunnel fibreux facilitant l'ablation du matériau et protégeant le muscle gastrique).

Par ailleurs, l'anneau gastrique ainsi décrit crée, par sa mise en place, une partition horizontale créant une configuration de l'estomac en deux parties ou poches, avec glissement de l'anneau due aux risques de dilatation de la poche inférieure.

On connaît également de FR-A-2 798 280, un anneau de gastroplastie dépourvu de ballon dilatable et réalisé sous la forme d'un tube pourvu d'un méplat d'appui contre la paroi gastrique. Cet anneau doit être maintenu en configuration fermée au moyen d'un fil de suture. Son diamètre en configuration fermé dépend de la tension donnée à la suture par le chirurgien, avec une reproductibilité non garantie.

On connaît enfin de EP-A-1 036 545 un anneau gastrique non dilatable pourvu d'une ouverture de passage d'une de ses extrémités en configuration fermée. Le verrouillage obtenu n'est pas optimal.

La solution mise en oeuvre, selon la demande, est de nature à répondre aux problèmes posés, à partir d'un concept de réalisation original et une méthode de pose qui garantit la position de l'anneau en place.

La solution visée met en oeuvre un procédé de pose spécifique du produit et qui a pour résultat l'obtention d'effets rapides sur l'obésité du patient.

Selon l'invention, un anneau gastrique dépourvu de partie dilatable est réalisé en un matériau élastomère notamment siliconé, présente une souplesse de déformabilité et comprend différentes zones distinctes successives conformées sur l'ensemble de sa longueur développée, tout en formant un ensemble monobloc et unitaire, ces zones incluant deux zones d'extrémité conformées spécifiquement pour constituer des parties mâle-femelle d'emboîtement souple et de verrouillage en position et, entre ces deux zones, une zone intermédiaire de raccordement à section pleine sur l'essentiel de sa longueur, destinée à être plus spécifiquement en simple appui sur la partie de l'estomac à enserrer. Cet anneau est remarquable en ce que les zones d'extrémité définissent, d'une part, une première zone de tête pourvue d'une patte profilée recourbée et, d'autre part, une seconde zone d'extrémité arrière d'ancrage formant nez principal, ces zones étant aptes à s'intégrer l'une dans l'autre, par introduction du nez de la zone d'ancrage dans une ouverture définie entre deux parois latérales reliant la patte à la zone intermédiaire.

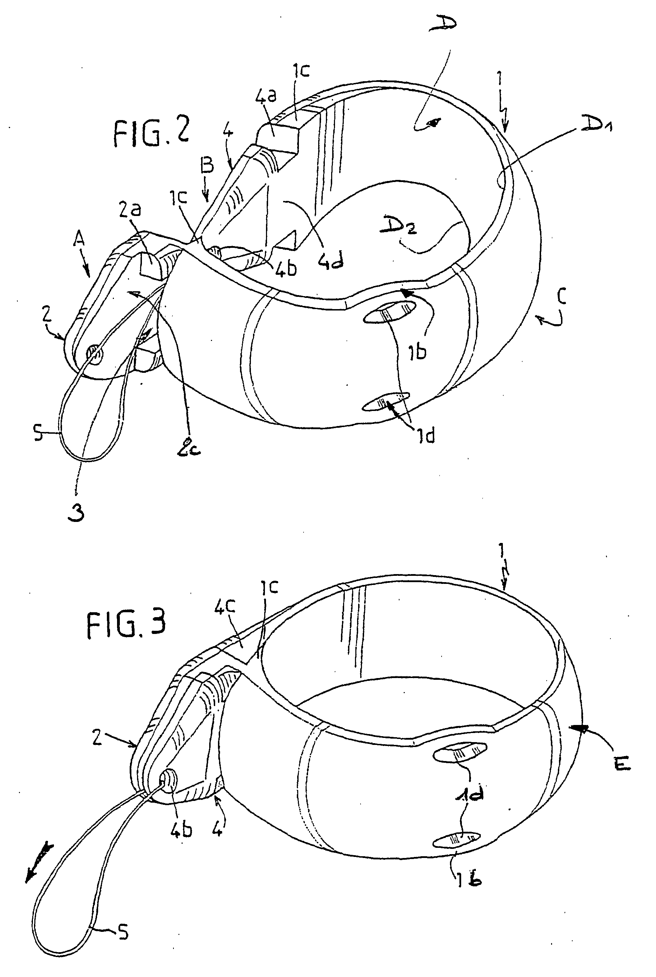

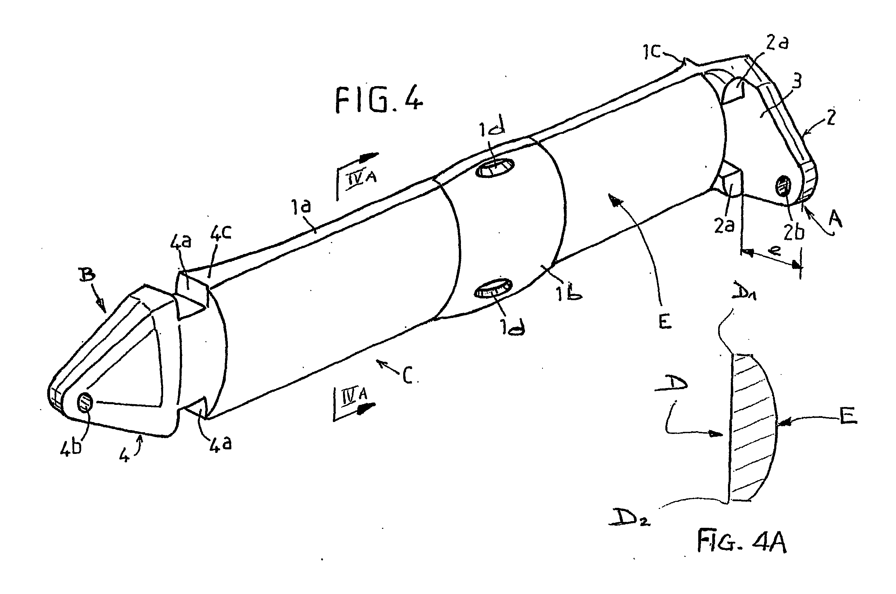

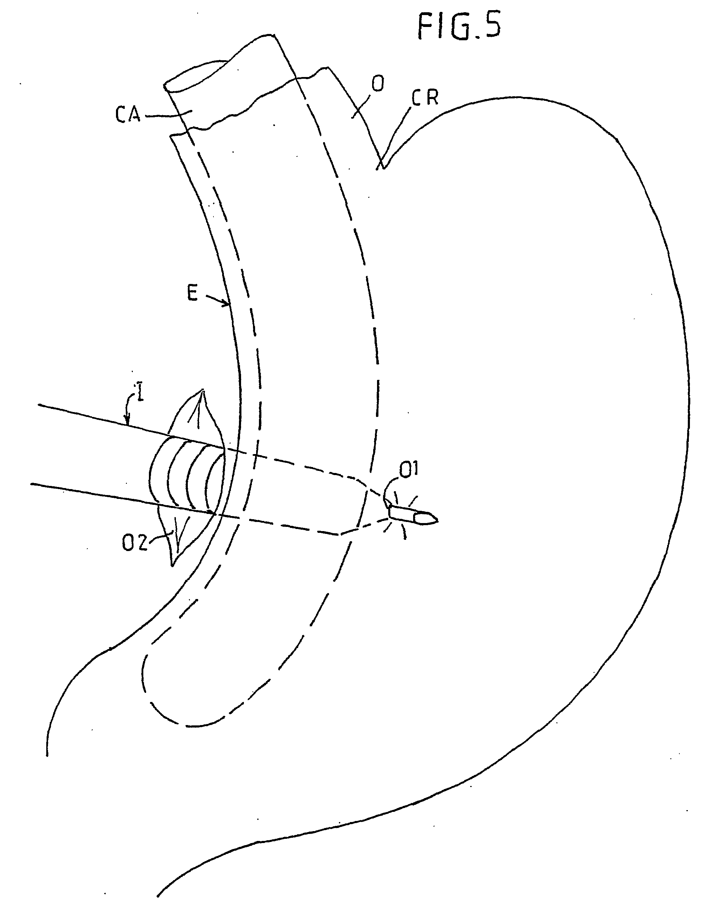

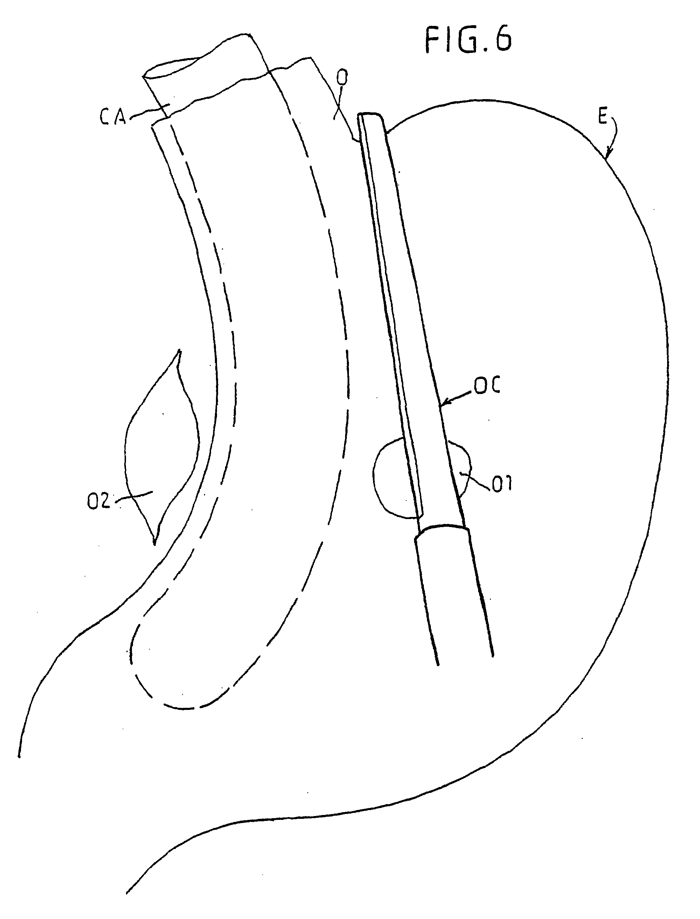

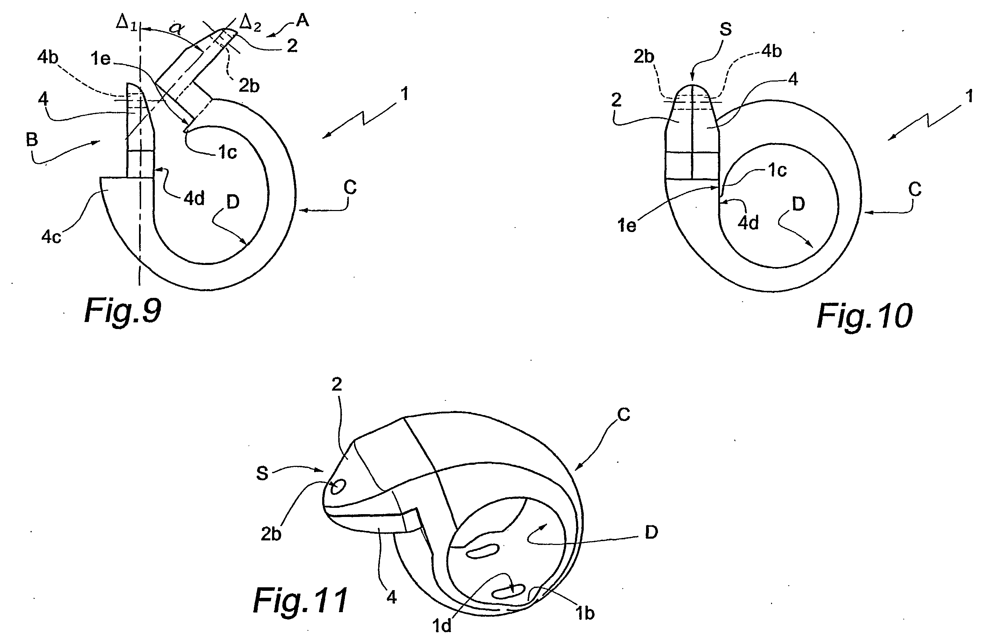

Afin de rendre plus concret son objet, l'invention est illustrée de manière non limitative aux figures des dessins où :
■ La figure 1 est une vue à caractère schématique illustrant, selon l'art antérieur, la pose d'un anneau gastrique,
■ La figure 2 est une vue en perspective avant serrage et fermeture de l'anneau, selon l'invention, l'anneau étant représenté en tant que tel et non positionné,
■ La figure 3 est une vue en perspective de l'anneau gastrique défini selon la figure 2, après fermeture.
■ La figure 4 est une vue en perspective de l'anneau représenté à plat déplié pour illustrer l'ensemble de ses caractéristiques.
■ La figure 4A est une coupe selon la ligne IV-IV à la figure 4,
■ Les figures 5, 6, 7 et 8 illustrent les différentes phases du procédé de mise en place de l'anneau autour de l'endroit spécifique de l'estomac.
■ La figure 9 est une vue de côté, avant utilisation, d'un anneau conforme à un second mode de réalisation de l'invention.
■ La figure 10 est une vue de côté de l'anneau de la figure 9 en configuration fermée, et
■ La figure 11 est une vue en perspective de l'anneau des figures 9 et 10 en configuration fermée.

Afin de rendre plus concret l'objet de l'invention, on le décrit maintenant d'une manière non limitative illustrée aux figures des dessins.

L'anneau gastrique selon l'invention est référencé dans son ensemble par 1 et est réalisé en un matériau élastomère moulé et monobloc. Il présente une certaine élasticité avec capacité de déformation. En position fermée, il présente une configuration en collier, entourant la partie souhaitée de l'estomac. Cet anneau est de préférence en matériau siliconé médical implantable, tel que celui connu dans 1e commerce sous la marque « NUSIL ». Ce matériau est radio opaque et biocompatible pour être implantable dans le corps humain en répondant ainsi aux exigences de compatibilité et sans être destructible. Cet anneau présente une mémoire de forme pour le ramener naturellement en position de collier avant fermeture, comme représenté figure 9 des dessins, ou sensiblement.

De par son matériau constitutif en élastomère et siliconé de préférence, il offre une certaine souplesse de déformabilité, de manière à permettre son introduction dans le lieu souhaité et d'être déformé pour constituer une configuration en collier et épouser au mieux les formes enserrées, en permettant un mode d'ancrage caractéristique de l'invention.

A l'état de mise en oeuvre, l'anneau est déployé sur toute sa longueur. A l'état de fermeture, il est replié à la manière d'un collier et selon l'invention, dans un montage et fixation réversible mais sécurisante.

L'anneau 1 est dépourvu de ballon dilatable, ce qui lui confère une fiabilité accrue par rapport aux dispositifs connus, notamment de WO-A-94/27504. II n'est donc pas réglable en diamètre.

Il est remarquable en ce qu'il comprend différentes zones distinctes successives conformées sur l'ensemble de sa longueur développée, tout en formant un ensemble monobloc et unitaire évitant des zones de faiblesse, telles qu'il peut exister dans les dispositifs à ballon dilatable. Les deux extrémités de l'anneau 1 sont conformées spécifiquement pour constituer des parties mâle-femelle d'emboîtement souple et de verrouillage en position en définissant une première zone de tête A et une seconde zone d'extrémité arrière d'ancrage B formant nez principal, ces zones s'intégrant l'une dans l'autre, ainsi qu'il sera précisé par la suite.

Entre les deux zones précitées, l'anneau comprend une troisième zone intermédiaire C de longueur et de raccordement. Cette zone est destinée à être plus spécifiquement en appui sur la partie de l'estomac à enserrer. La face interne D de l'anneau et les bords atraumatiques D₁ et D₂ sont lisses, ce qui est à rapprocher du fait qu'aucun ballon dilatable n'est prévu sur cette face.

Sur une partie de la zone C intermédiaire de raccordement, l'anneau présente un côté intérieur des formes en saillies 1b autorisant le contact en pression ponctuelle sur la partie d'estomac en regard, avec un effet anti-glissement. Ensuite, il est prévu dans cette partie la formation de deux cavités 1d dans l'épaisseur de l'anneau permettant l'enfilage de fils de ligaturage L complémentaires destinés à la fixation complémentaire de l'anneau par rapport à son environnement (paroi gastrique ou feutrage graineux), évitant son glissement et le basculement possible. La partie 1b est renflée par rapport au reste de la partie C et constitue des formes en saillie d'appui ponctuel complémentaire sur l'estomac, sans qu'il y ait d'adhésion à la paroi en regard de l'estomac.

La partie extérieure E apparente de l'anneau est sensiblement bombée sur toute sa longueur et dans un plan transversal.

Comme il ressort de la figure 4A, la partie C est à section pleine, sauf au niveau des cavités 1d. Ce caractère plein, c'est-à-dire monobloc, unitaire et massique, lui confère une résistance mécanique accrue et une bonne élasticité. En effet, le caractère plein de cette section garantit la stabilité dans le temps du calibrage obtenu avec l'anneau 1 qui, du fait de l'élasticité et de la mémoire de son matériau constitutif, reprend sa géométrie nominale après s'être éventuellement déformé au passage des aliments. Ce caractère plein induit également une homogénéité lors d'une déformation de l'anneau, en cours de mise en place ou une fois posé.

La zone de tête A est établie avec une patte profilée 2 qui est recourbée et disposée à l'extrémité avant du corps de l'anneau, en étant liée à la partie C par la jonction de deux parois latérales 2a, laissant apparaître entre elles une ouverture autorisant le passage et l'introduction et positionnement de l'autre extrémité en forme de nez 4.

La patte 2 est globalement plane et s'étend selon un plan globalement perpendiculaire à la direction principale de la partie C dans la configuration de la figure 4.

Plus spécifiquement, la patte principale 2 présente, en prolongement, des parois de liaison avec deux saillies parallèles 2a définissant un couloir 3 de passage de la seconde extrémité, en forme de nez 4. La patte principale présente, par ailleurs, des faces de butée, ainsi qu'il sera précisé par la suite. Cette extrémité ou patte 2 de l'anneau 1 présente une forme secondaire en nez, d'aspect triangulaire avec une face plate apparente de longueur e égale à la longueur de la zone de positionnement des butées. En extrémité, la patte perforée présente une ouverture transversale 2b pour le passage d'une boucle de traction 5.

Parallèlement, l'extrémité arrière 4 de l'anneau présente une forme principale en nez ayant un profil de dimension correspondant à celui de la forme en nez établie sur la patte avant. Entre la forme en nez 4 de la partie d'ancrage et la zone C, sont conformées deux échancrures 4a, profilées et opposées, dont la profondeur correspond en fait à l'épaisseur des saillies 2a établies sur la patte principale.

L'extrémité de la forme principale en nez 4 présente aussi une ouverture 4b pour le passage d'une ligature ou boucle de traction.

Ainsi on comprend, en se référant aux figures 2, 3, 7 et 8 que la fermeture de l'anneau gastrique s'effectue par introduction de la forme principale du nez de l'extrémité arrière 4 de l'anneau dans l'ouverture ou couloir 3 de passage situé dans la zone de raccordement de la patte avant et de l'extrémité avant de l'anneau.

La forme principale en nez arrière 4 est tirée par une boucle de traction 5 par le biais d'un instrument de passage qui l'amène à être déformée élastiquement pour traverser le passage d'ouverture 3 et s'ancrer sur la patte avant 2 après juxtaposition.

Les échancrures 4a précitées entourent alors les parties en saillie 2a de la patte avant 2, tandis que les formes principale et secondaire en forme de nez sont juxtaposées l'une contre l'autre.

Les ouvertures 2b et 4b se trouvent alors en regard l'une de l'autre, de sorte que la boucle de traction 5 peut aussi servir comme moyen de ligaturage et de fixation.

Dans le second mode de réalisation de l'invention représenté aux figures 9 à 11, les éléments analogues à ceux du premier mode de réalisation portent des références identiques.

Comme il ressort plus particulièrement de la figure 9, l'anneau 1 a, avant utilisation, et comme résultat de son procédé de fabrication par moulage, une forme ouverte dans laquelle le nez 4 prolonge sensiblement la zone intermédiaire C, selon une direction Δ₁, alors que la patte 2 s'étend globalement selon une direction Δ₂ faisant un angle α de l'ordre de 45° avec la direction Δ₁.

Comme précédemment, la surface interne D de la partie C est lisse, ce qui est à rapprocher du fait que l'anneau 1 est dépourvu de ballon dilatable et a une section pleine sur l'essentiel de la longueur de la partie C, à l'exception des zones 1b où sont formées les cavités 1d.

Lorsque l'anneau est fermé, comme représenté aux figures 10 et 11, la patte 2 et le nez 4 forment ensemble une partie en saillie S dont les bords extérieurs sont arrondis et qui est atraumatique. En outre, dans cette configuration, la face interne D de la zone C est sensiblement circulaire, ce qui permet un calibrage précis de l'estomac d'un patient.

En outre et selon une disposition complémentaire avantageuse, la face interne D de l'anneau présente du côté tête A un profil 1c en prolongement de la tête, orienté à l'opposé de celle-ci et dans un plan sous-jacent, de manière à constituer un plan d'appui 1e et de fiabilité de la partie 4d de l'anneau adjacente aux échancrures 4a. On obtient ainsi un effet de stabilité accrue et un recouvrement, par le profil 1c, de la zone de jonction entre les zones A et B, vu de l'intérieur de l'anneau. Les risques de blessure ou d'irritation de la paroi gastrique sont ainsi limités.

Par ailleurs, la section 4c de la partie de l'anneau ménagée au voisinage du nez 4 peut être plus épaisse pour assurer une meilleure tenue.

L'anneau gastrique ainsi réalisé est donc facile à mettre en place. Il est indémontable de lui-même, sauf à ce que l'opérateur déforme la partie du nez 4 de l'extrémité arrière B de l'anneau pour le retirer et le diriger vers l'ouverture d'introduction 3 formée sur la partie avant A de celui-ci. La boucle de traction peut servir à une fonction complémentaire de fixation par ligature si nécessaire après introduction dans les ouvertures 2b et 4b formées sur les parties nez de l'anneau. L'anneau offre une double sécurité par le verrouillage spécifique des parties de nez entre elles, de la patte et de l'extrémité arrière de l'anneau, d'une part, et, d'autre part, de la liaison échancrures-saillies 4a - 2a.

L'anneau ayant ainsi été défini dans ses caractéristiques structurelles, il convient dès lors d'exposer la méthode de pose et l'endroit de positionnement de l'anneau sur l'estomac en se référant aux figures 5 à 8 des dessins, et ce pour répondre aux effets recherchés de réduction gastrique pour traiter l'obésité.

Selon l'invention, il y a tout d'abord lieu d'introduire par la bouche du patient un tube gastrique dénommé tube de Faucher de 33 French (CA) arrivant dans l'estomac (E). Pour réduire le volume utile de l'estomac, il convient d'intervenir sur la partie d'estomac supérieure qui présente un orifice dénommé cardia (CR) ou s'abouche l'oesophage (O). Cet orifice délimite avec la partie supérieure de l'estomac une cavité de capacité plus ou moins importante chez les individus (figure 5). L'opérateur, à l'aide d'un instrument (I) va perforer la paroi d'estomac à un premier site (O1), entre 6 et 8 cm sous le cardia. Une seconde découpe sépare les deux parties gastriques dans un plan qui est vertical, et ce, à l'aide d'un instrument connu de l'homme de l'art.

Cette seconde découpe (O2) s'effectue proche du positionnement du tube gastrique dans l'estomac (figure 5). L'opérateur procède ensuite, à l'aide d'un outil de coupe (OC), à la partition verticale de l'estomac, de manière à créer une nouvelle poche gastrique près de la petite courbure gastrique et sera de petit volume 50 à 70 ml.

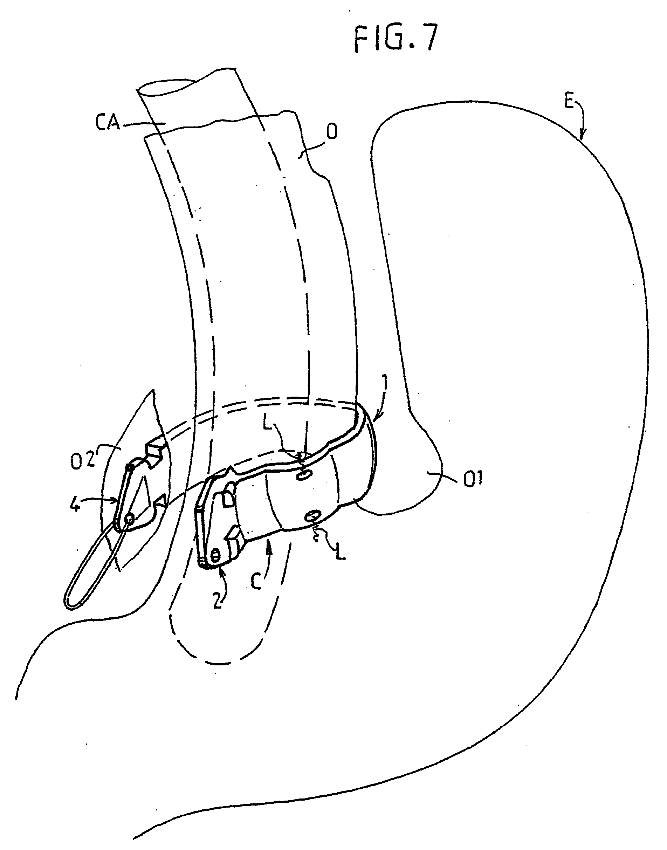

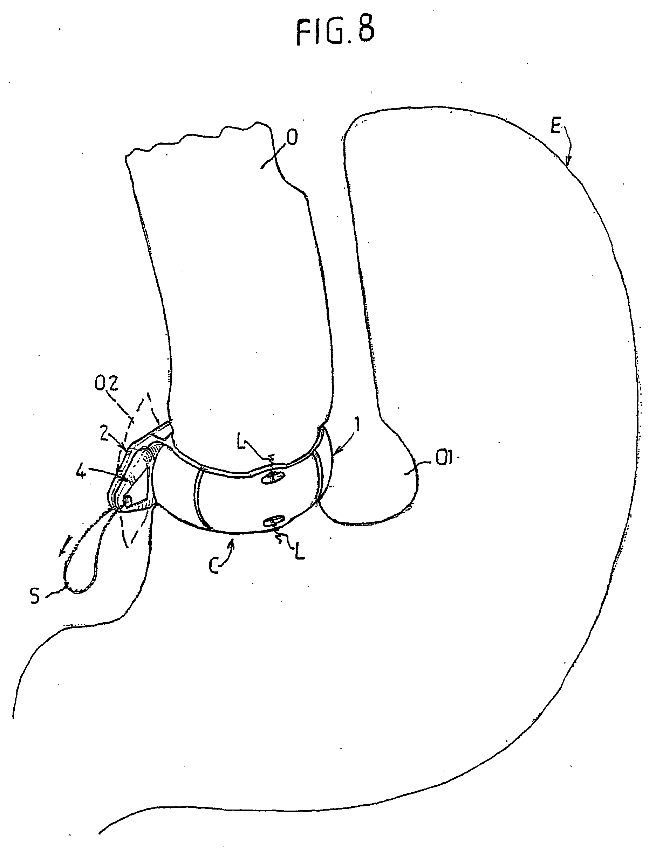

Sa vidange se fera par un orifice qu'il faudra calibrer. L'opérateur introduit l'anneau (1) qui se présente en position développée à l'aide d'un instrument de préhension complémentaire (figure 7). A l'aide d'un élément de préhension, l'opérateur va saisir la boucle de traction (5) et introduire l'extrémité arrière de l'anneau dans la partie patte avant d'ancrage, et assurer ensuite la fermeture, comme représenté figure 8 des dessins. Il suffit ensuite d'ajouter un ou deux ligaturages complémentaires (L) établis à partir des découpes formées dans la zone intermédiaire de l'anneau, ces points étant fixés sur l'environnement de l'estomac, le tube de calibrage de Faucher est ensuite retiré.

Les avantages de l'invention sont nombreux et on souligne en particulier :
- la qualité de l'anneau et de sa position sécurisée sur l'estomac,
- sa fiabilité de pose, son accessibilité aisée, et son démontage rapide.
Ses caractéristiques correspondent à la nécessité d'un calibrage invulnérable, non agressif pour la paroi gastrique évitant tout glissement et érosion. Cette intervention peut être réalisée en cas de hernie hiatale, pathologie qui est un écueil à l'utilisation d'un anneau gastroplastie pour partition horizontale.
Cette intervention est réversible et l'anneau peut être récupéré pour une nouvelle utilisation.

L'anneau est bien toléré de par sa constitution et ne risque pas d'entraîner d'inflammations locales indésirables. Il n'y a par ailleurs aucun risque de traumatisme, car l'anneau utilisé est unique sans autres accessoires comme cela est le cas souvent, selon l'art antérieur.

## Revendications

1. Anneau gastrique dépourvu de partie dilatable réalisé en un matériau élastomère notamment siliconé, présentant une souplesse de déformabilité et comprenant différentes zones distinctes successives conformées sur l'ensemble de sa longueur développée, tout en formant un ensemble monobloc et unitaire, lesdites zones incluant deux zones d'extrémité (A, B) conformées spécifiquement pour constituer des parties mâle-femelle d'emboîtement souple et de verrouillage en position et, entre ces deux zones, une zone intermédiaire (C) de raccordement, à section pleine sur l'essentiel de sa longueur, destinée à être plus spécifiquement en simple appui sur la partie de l'estomac à enserrer, **caractérisé en ce que** lesdites deux zones d'extrémités définissent, d'une part, une première zone de tête (A) pourvue d'une patte profilée (2) recourbée et, d'autre part, une seconde zone d'extrémité arrière d'ancrage (B) formant nez principal (4), lesdites zones (A, B) étant aptes à s'intégrer l'une dans l'autre, par l'introduction du nez (4) de la zone d'ancrage (B) dans une ouverture (3) définie entre deux parois latérales (2a) reliant ladite patte (2) à ladite zone intermédiaire (C).

2. Anneau selon la revendication 1, **caractérisé en ce que** sur une partie de la zone (C) intermédiaire de raccordement, l'anneau présente des formes en saillies (1b) autorisant le contact en pression ponctuelle sur la partie d'estomac en regard avec un effet anti-glissement, et **en ce qu'**il est prévu dans cette partie la formation de deux cavités (1d) dans l'épaisseur de l'anneau permettant l'enfilage de fils de fixation (L) complémentaires destinés à positionner l'anneau par rapport à son environnement et éviter sa bascule.

3. Anneau selon la revendication 1, **caractérisé en ce que** la partie extérieure (E) de la zone intermédiaire (C) de l'anneau (1) est sensiblement bombée sur toute sa longueur et dans un plan transversal.

4. Anneau selon l'une quelconque des revendications précédentes, **caractérisé en ce qu'**il présente une mémoire de forme pour le ramener sensiblement en position de collier avant fermeture.

5. Anneau selon l'une quelconque des revendications précédentes, **caractérisé en ce que** ladite patte principale (2) présente en prolongement des parois de liaison avec deux saillies parallèles (2a) définissant un couloir (3) de passage de la seconde extrémité en forme de nez (4), et **en ce que** la patte principale présente des faces de butée, ladite extrémité en patte de l'anneau présentant une forme secondaire en nez, d'aspect triangulaire avec une face plate apparente (2).

6. Anneau selon la revendication 5, **caractérisé en ce que** la patte perforée présente une ouverture transversale (2b) pour le passage d'une boucle de traction (5).

7. Anneau selon l'une quelconque des revendications précédentes, **caractérisé en ce que** l'autre extrémité arrière (4) de l'anneau présente une forme principale en nez ayant un profil de dimension correspondant à celui de la forme en nez établie sur la patte avant et **en ce que** à l'arrière de ladite forme en nez de la partie d'ancrage, sont conformées deux échancrures (4a) profilées opposées, dont la profondeur correspond à l'épaisseur des saillies (2a) établies sur la patte principale.

8. Anneau selon la revendication 7, **caractérisé en ce que** l'extrémité de la forme principale en nez présente une ouverture (4b) pour le passage d'une ligature ou boucle de traction.

9. Anneau selon l'une quelconque des revendications précédentes, **caractérisé en ce que** la face interne (D) de l'anneau présente du côté tête (A) un profil (1c) en prolongement de la tête orientée à l'opposé de celle-ci, de manière à constituer un plan d'appui (le) et de fiabilité de la partie d'anneau adjacente aux dites échancrures (4a).

10. Anneau selon l'une quelconque des revendications précédentes, **caractérisé en ce que**, avant fermeture et lorsque aucun effort extérieur n'est exercé sur l'anneau, ladite zone de tête (A) et zone d'ancrage (B) s'étendent selon deux directions (Δ₁, Δ₂) non parallèles (α).

11. Anneau selon l'une quelconque des revendications précédentes, **caractérisé en ce que**, en configuration fermée dudit anneau, sa face interne (D) est globalement circulaire.

12. Anneau selon l'une quelconque des revendications précédentes, **caractérisé en ce que** ladite zone de tête (A) et ledit nez principal (4) sont chacun pourvus d'une ouverture (2b, 4b) de passage d'une ligature ou d'une boucle de traction (5), lesdites ouvertures se trouvant en regard l'une de l'autre en configuration fermée dudit anneau (1).

13. Anneau selon l'une quelconque des revendications précédentes, **caractérisé en ce que**, lorsqu'il est fermé, ladite patte (2) et ledit nez (4) forment ensemble une partie en saillie (S) par rapport à ladite zone intermédiaire (C).

14. Anneau selon l'une quelconque des revendications précédentes, **caractérisé en ce que**, lorsqu'il est fermé, ladite patte (2) et ledit nez (4) sont globalement parallèles et en appui l'un contre l'autre.

15. Anneau selon l'une des revendications précédentes, **caractérisé en ce que** ladite patte (2) s'étend selon un plan globalement perpendiculaire à la direction principale de ladite zone intermédiaire (C) lorsque l'anneau est disposé à plat.

## Claims

1. Gastric ring consisting of a dilatable part made of an elastomer material, siliconised in particular, exhibiting deformability flexibility and comprising different successive distinct areas formed over the whole of its developed length, the whole forming a single and unitary block assembly, the said areas including two end areas (A, B) formed specifically so as to constitute the male-female parts of a flexible box arrangement and screwed into position and, between these two areas, an intermediate connection area (C), with a flat cross-section over most of its length, intended to be more specifically a simple support on the part of the stomach to be gripped, **characterised in that** the said two end areas define, on the one hand, a first head area (A) provided with a profiled curved foot (2) and, on the other, a second rear end anchor area (B) forming the main nose (4), said areas (A, B) being capable of integrating into one another by the introduction of the nose (4) of the anchoring area (B) into an aperture (3) defined between two side walls (2a) connecting said foot (2) to said intermediate area (C).

2. Ring according to Claim 1, **characterised in that**, on a part of the intermediate connection area (C), the ring exhibits waisted shapes (1b) allowing for the contact with point-by-point pressure on the part of the stomach opposite with an anti-sliding effect, and that provision is made in this part for the formation of two cavities (1d) in the thickness of the ring, allowing for the threading of supplementary fixing threads (L), intended to position the ring in relation to its surroundings and to avoid it tilting.

3. Ring according to Claim 1, **characterised in that** the outer part (E) of the intermediate area (C) of the ring (1) is appreciably cambered over its entire length and in a transverse plane.

4. Ring according to any of the foregoing claims, **characterised in that** it exhibits a shape memory to return it appreciably to the position of a collar before closure.

5. Ring according to any of the foregoing claims, **characterised in that** said main foot (2) exhibits an extension of the connecting walls with two parallel projections (2a) defining a passage corridor (3) from the second end in the form of a nose (4), and **in that** the main foot exhibits stop faces, said foot end of the ring exhibiting a secondary nose shape, of triangular appearance with an apparently flat face (2).

6. Ring according to Claim 5, **characterised in that** the perforated foot exhibits a transverse aperture (2b) for the passage of a traction loop (5).

7. Ring according to any of the foregoing claims, **characterised in that** the other rear end (4) of the ring exhibits a main shape of a nose with a dimensional profile corresponding to that of the shape of the nose formed on the front foot, and that the rear of said nose shape of the anchoring part, conform with two opposed profiled cut-outs (4a), the depth of which corresponds to the thickness of the projections (2a) located on the main foot

8. Ring according to Claim 7, **characterised in that** the end of the nose shape exhibits an aperture (4b) for the passage of a ligature or traction loop.

9. Ring according to any of the foregoing claims, **characterised in that** the internal face (D) of the ring exhibits on the head side (A) a profile (1c) in extension of the head oriented opposite to it, in such a way as to form a support and back-up plane (1e) for the part of the ring adjacent to said cut-outs (4a).

10. Ring according to any of the foregoing claims, **characterised in that**, before closure and while no external force is being applied to the ring, said head area (A) and anchoring area (B) are extended in two non-parallel (α) directions (Δ₁, Δ₂).

11. Ring according to any of the foregoing claims, **characterised in that**, in the closed configuration of the ring, its internal face (D) is overall circular.

12. Ring according to any of the foregoing claims, **characterised in that**, in said head area (A) and said main nose (4) are each provided with an aperture (2b, 4b) for the passage of a ligature or traction loop (5), said apertures being located opposite one another in the closed configuration of said ring (1).

13. Ring according to any of the foregoing claims, **characterised in that**, when it is closed, said foot (2) and said nose (4) together form a part (S) which projects in relation to said intermediate area (C).

14. Ring according to any of the foregoing claims, **characterised in that**, when it is closed, said foot (2) and said nose (4) are overall parallel and support one another.

15. Ring according to any of the foregoing claims, **characterised in that**, when it is closed, said foot (2) extends along a plane which is overall perpendicular to the principal direction of said intermediate area (C) when the ring is laid flat.

## Patentansprüche

1. Magenring ohne ein dehnbares Teil, der aus einem Elastomermaterial, insbesondere Silikonelastomer, das eine Verformungsnachgiebigkeit aufweist, hergestellt ist und unterschiedliche Zonen umfasst, die auf der Gesamtheit seiner abgewickelten Länge deutlich und aufeinanderfolgend ausgebildet sind, wobei sie eine einstückige und unitäre Anordnung bilden, und die Zonen zwei Endzonen (A, B), die spezifisch ausgebildet sind, um männlich-weibliche weiche Bestandteile zum Verschließen und Verriegeln in Position zu bilden und zwischen diesen zwei Zonen eine Verbindungszwischenzone (C) einschließen, die einen vollen Querschnitt über im Wesentlichen ihre gesamte Länge aufweist und dazu dient, ganz besonders in Flächenabstützung auf dem einzuspannenden Bereich des Magens zu sein,
**dadurch gekennzeichnet,**
**dass** die zwei Endzonen einerseits eine erste Kopfzone (A), die mit einem gekrümmten profilierten Lappen (2) versehen ist, und andererseits eine zweite hintere Endzone (B) zur Verankerung, die die Hauptnase bildet, definieren, wobei die Zonen (A, B) geeignet sind, durch Einführen der Nase (4) der Verankerungszone (B) in eine Öffnung, die zwischen zwei, den Lappen (2) mit der Zwischenzone (C) verbindenden Seitenwänden (2a) gebildet wird, ineinander eingesetzt zu werden.

2. Ring nach Anspruch 1, **dadurch gekennzeichnet, dass** auf einem Bereich der Verbindungszwischenzone (C) der Ring vorspringende Formen (1b) aufweist, die einen punktuellen Druckkontakt auf den gegenüberliegenden Teil des Magens mit einer Anti-Rutschwirkung zulassen, und dass in diesem Bereich die Bildung von zwei Aushöhlungen (1d) in der Dicke des Ringes vorgesehen ist, die das Einfädeln von zwei komplementären Befestigungsfäden (L) gestatten, die zur Positionierung des Ringes in Bezug auf seine Umgebung und zur Vermeidung seines Kippens dienen.

3. Ring nach Anspruch 1, **dadurch gekennzeichnet, dass** der Außenbereich (E) der Zwischenzone (C) des Ringes (1) merkbar auf seiner gesamten Länge und in einer Querebene ballig ist.

4. Ring nach einem der vorhergehenden Ansprüche, **dadurch gekennzeichnet, dass** er ein Formengedächtnis aufweist, um ihn vor seinem Schließen deutlich in eine Reifenstellung zu bringen.

5. Ring nach einem der vorhergehenden Ansprüche **dadurch gekennzeichnet, dass** der Hauptlappen (2) in Verlängerung zwei Verbindungswände mit zwei parallelen Vorsprüngen (2a) aufweist, die einen Durchgang (3) für das zweite Ende in Form einer Nase (4) begrenzen, und dass der Hauptlappen Anschlagflächen aufweist, wobei das lappenförmige Ende des Ringes eine Nasensekundärform von dreieckigem Aussehen mit einer flachen sichtbaren Vorseite (2) aufweist.

6. Ring nach Anspruch 5, **dadurch gekennzeichnet, dass** der perforierte Lappen eine Queröffnung (2b) für den Durchgang einer Zugschleife (5) aufweist.

7. Ring nach einem der vorhergehenden Ansprüche, **dadurch gekennzeichnet, dass** das andere hintere Ende (4) des Ringes eines Nasenhauptform aufweist, die ein Profil mit einer Abmessung aufweist, die der an dem vorderen Lappen vorgesehenen Nasenform entspricht, und dass hinter der Nasenform des Verankerungsteils zwei entgegengesetzt profilierte Ausschnitte (4a) ausgebildet sind, deren Tiefe der Dicke der an dem Hauptlappenvorgesehenen Vorsprünge (2a) entspricht.

8. Ring nach Anspruch 7, **dadurch gekennzeichnet, dass** das Ende der Nasenhauptform eine Öffnung (4b) für den Durchgang einer Ligatur oder Zugschleife aufweist.

9. Ring nach einem der vorhergehenden Ansprüche, **dadurch gekennzeichnet, dass** die Innenseite (D) des Ringes an der Kopfseite (A) in Verlängerung zum Kopf, entgegengesetzt zu ihm, ein Profil (1c) aufweist, um eine Ebene zum Abstützen (1e) und für die Zuverlässigkeit des Teils des Ringes benachbart zu den Ausschnitten (4a) zu bilden.

10. Ring nach einem der vorhergehenden Ansprüche, **dadurch gekennzeichnet, dass** vor dem Schließen und wenn keine äußere Kraft auf den Ring ausgeübt wird, die Kopfzone (A) und die Verankerungszone (B) sich in zwei nicht parallele (α) Richtungen (Δ₁, Δ₂) erstrecken.

11. Ring nach einem der vorhergehenden Ansprüche, **dadurch gekennzeichnet, dass** in der geschlossenen Stellung des Ringes seine Innenfläche (D) im Ganzen gesehen kreisförmig ist.

12. Ring nach einem der vorhergehenden Ansprüche, **dadurch gekennzeichnet, dass** die Kopfzone (A) und die Hauptnase (4) jeweils mit einer Öffnung (2b, 4b) für den Durchgang einer Ligatur oder einer Zugschleife (5) versehen sind, wobei die Öffnungen sich in geschlossenem Zustand des Ringes (1) einander in Gegenüberstellung befinden.

13. Ring nach einem der vorhergehenden Ansprüche, **dadurch gekennzeichnet, dass**, wenn er geschlossen ist, der Lappen (2) und die Nase (4) zusammen einen vorspringenden Bereich (S) in Bezug auf die Zwischenzone (C) bilden.

14. Ring nach einem der vorhergehenden Ansprüche, **dadurch gekennzeichnet, dass**, wenn er geschlossen ist, der Lappen (2) und die Nase (4) im Wesentlichen parallel und in wechselseitiger Abstützung sind.

15. Ring nach einem der vorhergehenden Ansprüche, **dadurch gekennzeichnet, dass** der Lappen (2) sich entsprechend einer im Wesentlichen senkrechten Ebene in Bezug auf die Hauptrichtung der Zwischenzone (C) erstreckt, wenn der Ring flach angeordnet ist.
